# EUROPEAN PATENT APPLICATION

(11) **EP 1 900 344 A2**
(43) Date of publication of application: **19.03.2008**
(21) Application number: 06380246.6
(22) Date of filing: 15.09.2006
(51) Int. Cl.: A61F 2/36, A61F 2/30

(54) **Resurfacing femoral head component**

(71) Applicant: Corin Limited, Cirencester Gloucestershire GL7 1YJ (GB)
(72) Inventor: Collins, Simon, Gloucester GL8 8NT (GB); Emslie, Ian, Cirencester GL7 1DY (GB); Royle, Matthew James, Lancashire FY6 7NA (GB); Bauer, Andre, 29690 Casares, Malaga, NIE X 1715148 S (ES)
(74) Representative: McLeish, Nicholas Alistair Maxwell

(57) **Abstract**

A hip resurfacing femoral component (110) is designed to be used with a prepared femur. The hip resurfacing femoral component (110) comprises a head portion (112) which has a substantially spherical outwardly facing bearing surface (111) with an attachment surface (119) opposing the bearing surface (111) of the head portion (112). The femoral component (110) further comprises a skirt portion (121) with an outer skirt surface (124) which depends from the substantially spherical bearing surface (111) and an inner skirt surface (123). The inner skirt surface (123) and the attachment surface (119) are configured to fit over the prepared femur.

## Description

The present invention relates to a femoral head component for use in hip resurfacing procedures.

Conventional total hip replacement (THR) in a procedure for the treatment of arthritis of the hip, a condition which causes considerable pain and loss of movement. As is well known, the hip is a ball and socket joint which allows the upper leg to move from side to side, back to front, and to rotate. The joint is made up of the head of the femur (the ball) which fits into the acetabulum (the socket). In a healthy hip, both the head of the femur and acetabulum are covered with cartilage which provides a smooth surface allowing the joint to move freely.

In conventional total hip (THR) the worn head is cut off the femur and replaced with a metal ball and stem in the shaft of the femur and a plastic cup is placed in the prepared acetabulum. Both elements are typically anchored to the bone by "bone cement". This has become a very common surgical procedure with some 45,000 hip replacements being carried out in the UK each year.

The plastic used to form the cup is inert and so is well tolerated by the body. Nevertheless, as the metal ball rubs against the plastic cup, tiny particles of the plastic are worn away. This plastic debris causes an irritation. Furthermore, as the particles get between the bone and the artificial joint, this irritation causes surrounding bone to be absorbed by the body, leading to a loosening of the artificial joint. In older people with a lower activity level, this may not happen for 20 or more years, but in younger, more active patients, this may happen much sooner.

To overcome these problems in younger, more active patients requiring hip replacement, a different type of implant was needed. In 1991 a procedure for metal-on-metal (MoM) resurfacing of the hip was proposed. This had two major differences from a conventional THR. The first difference is that both components are typically made from metal, preferably cobalt chrome. By eliminating the plastic cup of a conventional THR, and making both parts of the bearing surface of metal, the resurfaced hip is expected to last much longer and therefore to be more suitable for higher demanding patients. The second difference is that the procedure is very bone conserving, since the head of the femur is simply reshaped and "resurfaced" rather than being removed.

However, a problem that exists with the hip resurfacing is the lack of ability to deal with options relating to offset of the bearing surface of the femoral head component and thereby the possibility for altering the leg length of a patient when carrying out a hip resurfacing operation.

In THR there were originally very few options relating to the offset of the femoral head and the associated change in leg length after operation. The introduction of modular heads in THR, where the surgeon is able to select heads with a different neck length ranging from approximately minus 4 mm (in relation to the standard neck length) up to plus 20 mm allowed for leg length of patients to be altered to optimise the biomechanics following the operation. Although a definitive range of acceptable and unacceptable leg length discrepancy after THR has never been established it is agreed by surgeons that it is advantageous to have equal, or nearly equal, leg length following a replacement hip operation. In particular, it is noted that the normal process of degeneration and loss of cartilage during osteoarthritis of the hip leads to some shortening of the effective limb, as does unilateral dysplasia, in which leg length differences of up to 5 cm can occur. In the latter case some of the discrepancy may be corrected by correct acetabular placement, but the ability to alter leg length using modular heads available when carrying out a THR is also very useful.

In contrast, in hip resurfacing, the operative technique is bone preserving and important structures such as the femoral head and neck are preserved and simply prepared to receive the new bearing surface. Therefore, the standard hip resurfacing component known in the prior art provides no opportunity to significantly modify the offset of the bearing surface and thereby the post operative leg length desired cannot generally be achieved. However, some shortening of a too long limb may be feasible by virtue of seating the femoral component deeper below the juncture in the femoral head and neck, this is however limited and can produce overhang of the metal component on the femoral neck leading to inadequate fixation.

Therefore, it is desired to provide an improved femoral component for use in the hip resurfacing procedure which allows for alteration to leg length during the surgical procedure.

### SUMMARY OF THE INVENTION

According to the present invention there is provided a first aspect of a hip resurfacing femoral component for use with a prepared femur comprising: a head portion having a substantially spherical outwardly facing bearing surface; an attachment surface opposing said bearing surface of the head portion; and a skirt portion having an outer skirt surface depending from said substantially spherical bearing surface and an inner skirt surface, the inner skirt surface and the attachment surface being configured to fit over the prepared femur.

This is advantageous since the hip resurfacing femoral component is offset relative to the femur by virtue of the skirt portion. This thereby allows for post operative leg lengthening thereby providing for the legs to have equal, or nearly equal, lengths following the resurfacing procedure.

According to a second aspect of the present invention there is provided a hip resurfacing femoral component for use with a prepared femur and having a distally extending longitudinal axis, said femoral component comprising, a head portion having a substantially spherical distally facing bearing surface, having a centre and a distal-most point of the bearing surface; and a proximally facing attachment surface opposing said bearing surface of the head portion and having a distal most point of the attachment surface; an offset distance being defined as the distance parallel to the axis from said distal most point of the attachment surface to said distal most point of said bearing surface; said femoral head being designed such that a ratio of the offset distance to the diameter of the substantially spherical bearing surface is greater than 0.25.

This embodiment is advantageous in that it also provides an offset of the hip resurfacing femoral component, thereby allowing for post operative leg lengthening. Preferably the longitudinal axis passes through the centre of the bearing surface. Advantageously the hip resurfacing femoral component further comprises a skirt portion depending from the spherical bearing surface to provide a greater range of motion of the joint.

Preferably the hip resurfacing femoral component further comprises a stem portion depending from the attachment surface. The stem portion may be formed with the head portion or attached thereto. For example the stem may be attached to the head portion with adhesive or molded thereto or may be attached by mechanical fixation using, for example, a taper fit, bayonet fitting or screw thread fixation. The stem portion helps to ensure correct alignment of the femoral component and provides post operative stability in the months following the operation.

Preferably the resurfacing femoral component has an insert portion providing the attachment surface and comprising a head portion engagement surface opposite the attachment surface for engagement with an insert portion engagement surface of the head portion which is opposite the bearing surface. The insert portion may be attached to the head portion by bonding, with adhesive or by the two portions being molded/fused together. Alternatively the insert portion and head portion may be mechanically attached by, for example, taper fit, a bayonet fitting, screw thread engagement or a push fit. The distance the insert portion is advanced into the head portion may be varied to allow for a variable offset of the hip resurfacing femoral component. The insert portion may hold a separate skirt portion in position between the head portion and insert portion or may be formed to include a skirt portion. A resurfacing femoral component comprising a separate head portion and insert portion, and skirt portion if desired, provides advantages including that the two components may be made of different materials to provide cost and/or weight savings.
Furthermore the modular device is more easy to manufacture and requires a reduced operative inventory.

In order to provide even more modularity, and thereby more options for the surgeon, the resurfacing femoral component preferably further comprises a spacer, which may be selected to give the desired offset. The spacer is positioned between the head portion engagement surface and insert portion engagement surface. The spacer can be made of many shapes, for example a simple cuboid or a more complex shape complimentary to the head and insert engagement surfaces, and may be attached to one of the head or insert engagement surfaces to allow for easier assembly of the hip resurfacing femoral component.

Advantageously the insert, and spacer if provided, have a throughbore provided therein. A bore is then provided in the head portion and a stem portion is provided with a projection extending from an upper surface thereof. The projection then extends through the throughbore(s) and into the bore in the head portion to easily assembly the hip resurfacing femoral component. Alternatively a projection may extend from the head portion, extend through the throughbore(s) and into a bore provided in the stem portion.

The resurfacing femoral component, or any portions thereof, are preferably made of metal or ceramic. To aid in implantation the attachment surface, and stem if applicable, may be coated with an osteo-conductive coating.

According to the present invention there is also provided a method of implanting the resurfacing femoral component in any of the forms described. The femoral component is assembled, if necessary. The femur is prepared to receive the component and the component is then positioned in place on the femur.

### Brief Description of the Drawings

Preferred embodiments of the invention will now be described, by way of example, with reference to the accompanying drawings, in which:
Figure 1 shows a side view of a resurfacing femoral head component of the prior art;
Figure 2 shows a view of a resurfacing femoral head component of Figure 1 with the interior features shown as dashed lines;
Figure 3a shows a cross-section through a longitudinal plane of a femoral head component according to an embodiment of the present invention;
Figure 4a shows a femoral head component according to the present invention in position on a prepared femur;
Figure 4b shows a section through the femoral head component and femur of Figure 4a;
Figure 5a shows a prior art femoral head component in position on a prepared femur;
Figure 5b shows a section through the femoral head component and femur of Figure 5a;
Figure 6 shows a femoral head component according to the present invention without a stem portion, indicating an offset distance of the component;
Figure 7a shows a cross-section through a longitudinal plane of a femoral head component according to an alternative embodiment of the present invention;
Figure 7b shows a cross-section through a longitudinal plane of a femoral head component according to another alternative embodiment of the present invention.
Figure 8 shows a cross-section along a longitudinal plane of a femoral head component according to a yet further embodiment of the present invention;
Figure 9 shows a cross-section along a longitudinal plane of a femoral head component according to a further embodiment of the present invention;
Figure 10 shows a cross-section along with a longitudinal plane of a femoral head component according to a yet further embodiment of the present invention; and
Figure 11 shows a cross-section along a longitudinal plane of a femoral head component according to a further embodiment of the present invention.

### Detailed Description of the Drawings

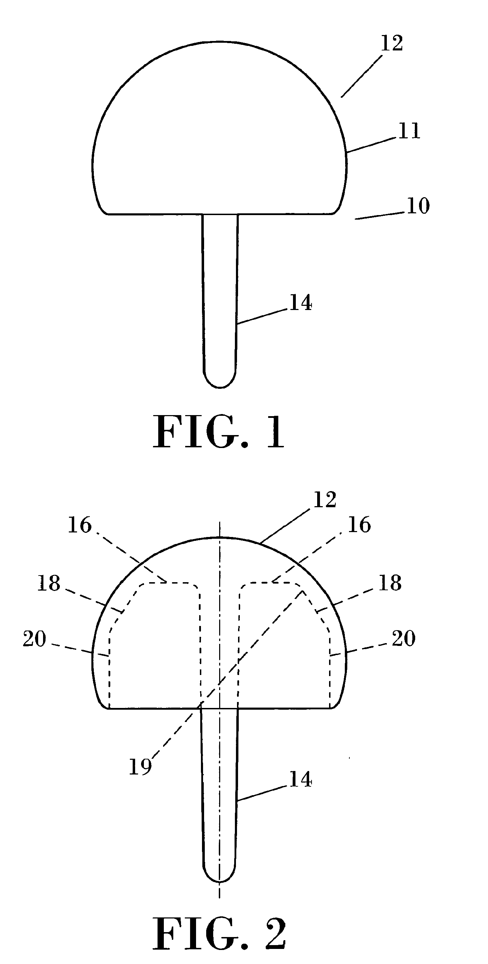

A conventional resurfacing femoral component 10 is shown in Figures 1 and 2 and comprises a head portion 12 having a bearing surface 11, which is a substantially spherical portion (generally slightly greater than a hemisphere), and a stem 14. In use, the stem 14 is positioned within the femur and is used to ensure that the femoral head is correctly positioned whilst giving post-operative stability in the first few months after implantation of the implant. As shown in Figure 2, at its upper end the stem 14 merges with a radially outwardly extending top inner surface 16 which in turn merges with a radially outwardly and downwardly depending upper inner surface 18, which in turn merges with a downwardly depending lower inner surface 20. The lower inner surface then merges with the bearing surface 11. Together the top inner surface 16, the upper inner surface 18 and the lower inner surface 20 form an inner surface 19 which fits over the prepared head of the femur. Together the bearing surface 11, lower inner surface 20, upper inner surface 18 and top inner surface 16 form the head portion 12. The resurfacing femoral component 10 is chosen by the surgeon to suit each individual patient based on the most suitable diameter of the bearing surface.

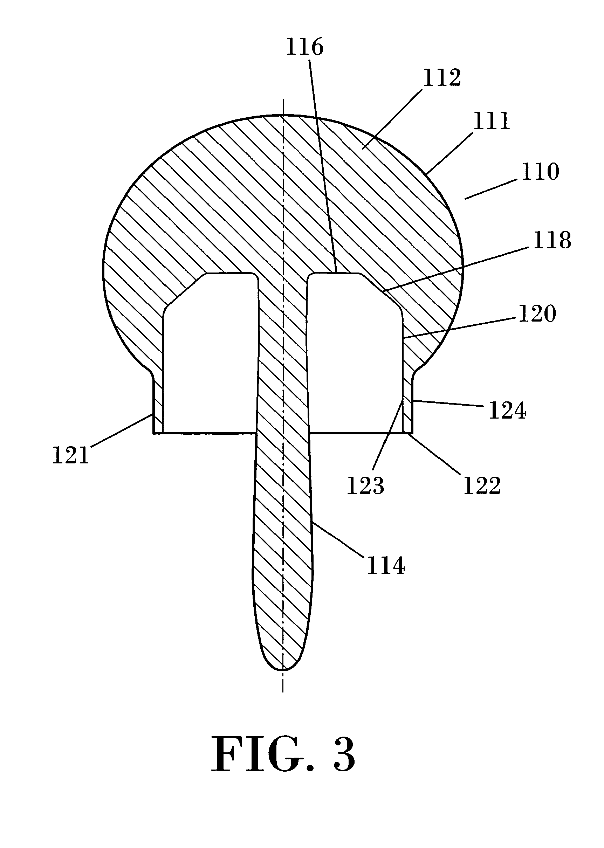

Figure 3 shows an embodiment of the present invention in which a femoral component 110 comprises a head portion 112 with an outwardly facing bearing surface 111 and a stem 114 as in the prior art. The femoral component having a longitudinal axis L. Like parts to the prior art component are given the same reference numeral with a different stem.

The embodiment differs from the prior art resurfacing head component in that the femoral head component 110 further comprises a skirt 121 which depends from the termination of the bearing surface 111. The skirt 121 comprises an inner skirt surface 123 which merges at one end with a lower inner surface 120 and at the opposing end with a radially outwardly extending wall 122. The wall 122 in turn merges with an upwardly extending outer skirt surface 124. The outer skirt surface 124 in turn merges with the bearing surface 111. The inner skirt surface 123, wall 122 and upwardly extending outer skirt surface 124 preferably form a cylinder, but may be ovoid or elliptical in shape. The femoral head component 110 further comprises a top inner surface 116, an upper inner surface 118 and lower inner surface 120 similarly to the device shown in Figure 3. Together the top inner surface 116, upper inner surface 118, lower inner surface 120 and inner skirt surface 123 form an inner surface 119 which fits over the prepared head of the femur.

The femoral component 110 may be made as a single piece or the skirt 121 may be attached, for example using electron beam welding. The skirt portion could also be an elongate cylinder which is attached, for example with adhesive or bonding to either the lower inner surface 120, or a recess therein. It is also envisaged that splines extending upwardly from the skirt may be fixed into keys provided in the lower inner surface 120.

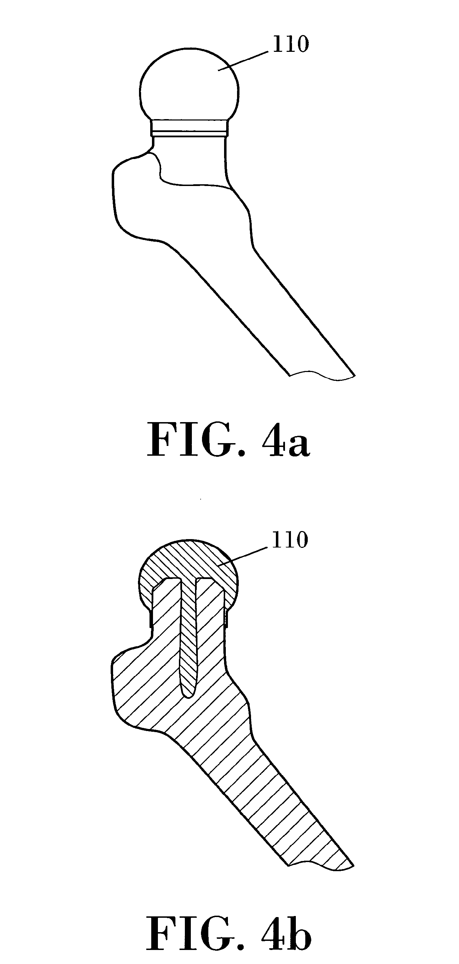

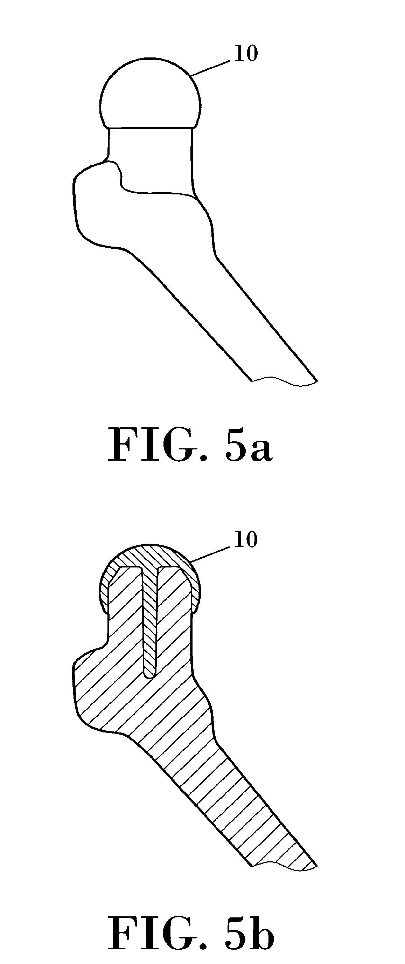

Figures 4a and 4b show a femoral head component, for example 110, of the present invention in position on a prepared femur. Figures 5a and 5b show a prior art femoral head component, for example 10, in position on a prepared femur. Comparing Figures 4 and 5 it can clearly be seen that the femoral head component of the present invention allows for an offset of the bearing surface from the femur. This offset gives the opportunity for surgeons to alter the post-operative leg length as desired.

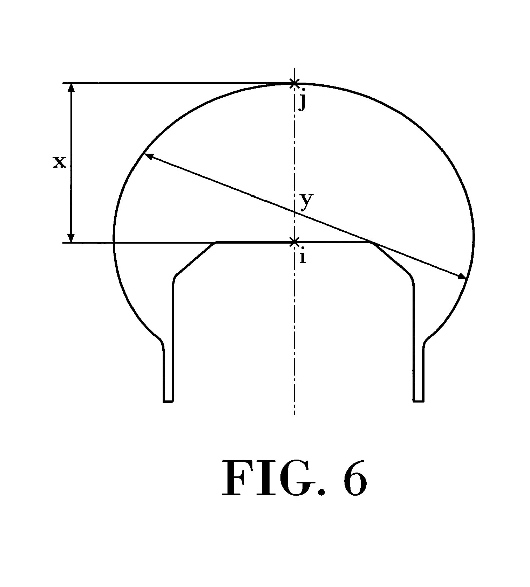

As shown in Figure 6 an offset distance x can be defined as the distance between a distal point i of the inner surface 119 on the longitudinal axis and a distal point j of the bearing surface 112 on the longitudinal axis. The ratio of the offset distance to the diameter of the bearing surface y is preferably greater than 0.25 and preferably lies in the range 0.25 to 0.75, more preferably in the range 0.28 to 0.66. This is in contrast to prior art resurfacing femoral head components in which the ratio is generally less than 0.2.

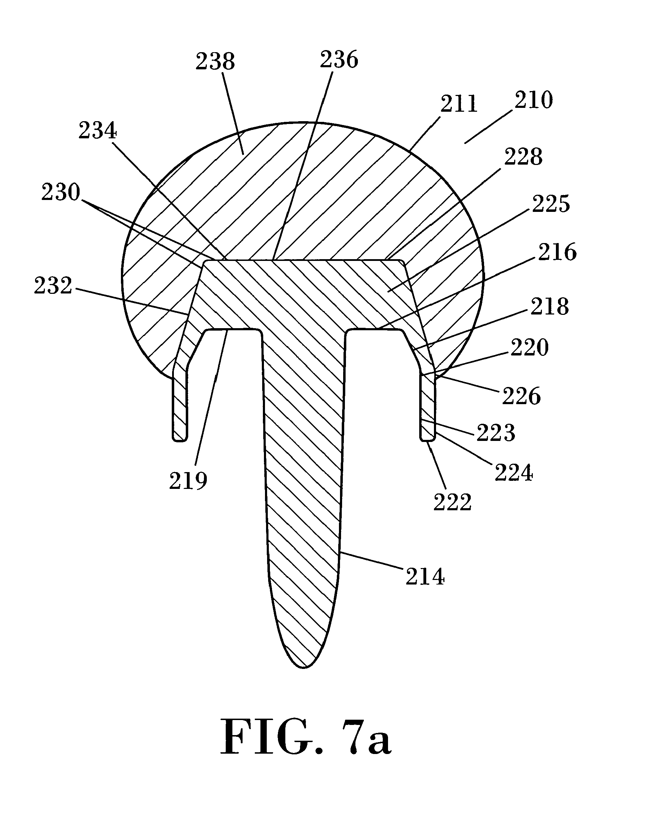

Figure 7a shows an alternative embodiment of the present invention in which a resurfacing femoral component 210 comprises two parts. The first part is an insert 225 which comprises a stem 214 which merges at its upper end with a radially outwardly extending top inner surface 216, which in turn merges with a radially outwardly and downwardly extending upper inner surface 218, which in turn merges with a downwardly depending lower inner surface 220. The lower inner surface 220 in turn merges with an inner skirt surface 223. The inner skirt surface 223 then merges with an outwardly extending wall 222, which in turn merges with an upwardly extending outer skirt surface 224. The inner skirt surface 223, wall 222 and outer skirt surface 224 form a skirt 221. The outer skirt surface 224 then merges with an upwardly extending outer insert surface 226, shown as being tapered but not limited thereto, which in turn merges with a top insert surface 228 of the insert 225. The outer insert surface 226 and upper insert surface 228 together form an engagement surface 230. The bearing surface 211 of the femoral head component itself merges with an upwardly extending inner wall 232 which in turn merges with a radially inwardly extending surface 234 to form an insert receiving recess 236. The bearing surface 211 and insert receiving recess 236 form the outer boundary of an upper femoral head component 238.

The insert receiving recess 236 and the engagement surface 230 of the insert 225 may be correspondingly frustoconical shaped to provide a taper fit between the insert 225 and upper femoral head component 238. Alternatively, the outer insert surface 226 may be threaded and the inner wall 232 of the insert receiving recess 236 may be provided with corresponding threads, such that the insert 225 and upper femoral head component 238 may be attached together by use of the screw threads. In a further alternative, the insert 225 may be fitted into the upper femoral head component 238 by use of a bayonet fitting or adhesive or may be molded thereto. In a further alternative the insert 225 may be press or push fit into the insert receiving recess 236, if push fit then splines may be provided on one of the insert 225 or the insert recess 236 to prevent rotation of the insert 225 within the insert recess 236.

The advantages of using a two part device as described above include that the two components, the insert 225 and upper femoral head component 238, may be made of different materials, leading to cost and/or weight savings. In addition, it is not necessary to provide the insert 225 with the same surface finish requirements as the bearing surface 211, thereby rendering the two-part device easier to manufacture. Since, in contrast to a one-piece device, the outer skirt surface 224 can be finished separately to the bearing surface 211 the manufacture process is also greatly simplified.

By varying the relative position of ball and insert it is possible to vary the length of the skirt 221 and thereby the offset of the bearing surface 211, resulting in greater interoperative flexibility for the surgeon when wishing to alter postoperative leg length whilst minimising the inventory of surgical component.

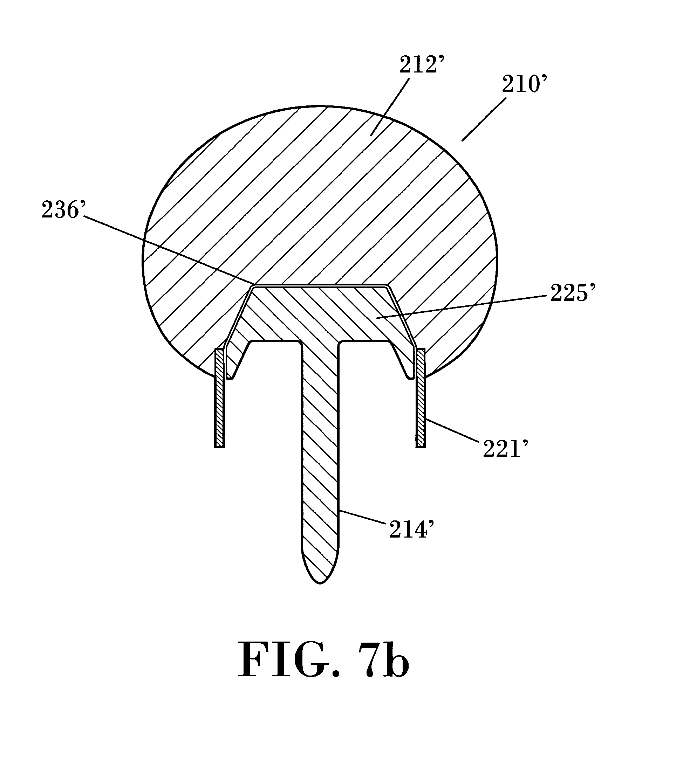

In an alternative embodiment shown in Figure 7b an insert 225' is provided similarly to the embodiment of Figure 7a. However, in the embodiment of Figure 7b the insert 225' is provided as a separate component to a skirt 221'. In this embodiment the skirt 221' is inserted part way into an insert receiving recess 236' and is held in place by the introduction into the insert receiving recess 236' of the insert 225'. The insert 225' may be held in place and thereby secure the skirt 221' in place, by a variety of attachment mechanisms, for example, a taper fit, screw-thread attachment, press or push fit, bayonet fitting or adhesive, bonding or moulding.

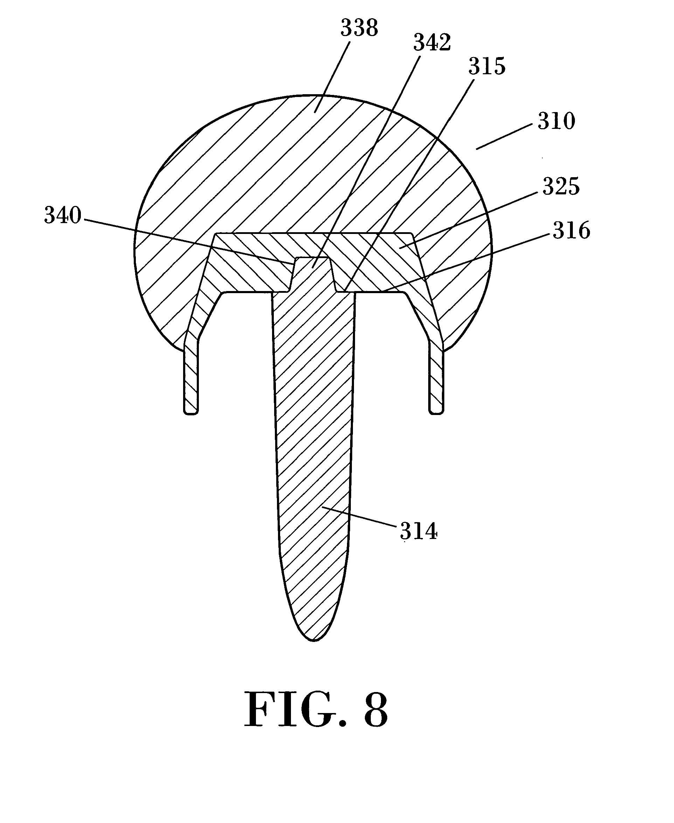

Figure 8 shows a further alternative embodiment of a femoral head component 310 comprising an upper femoral head component 338, insert 325 and stem 314 similarly to the device shown in Figure 7a. However, in the embodiment of Figure 8 the insert 325 comprises a top inner surface 316 which merges with an attachment portion 340 which extends into the insert 325 to form a bore.

The attachment portion 340 may take the form of a frustoconical shaped bore 340 for engagement with a frustoconical attachment member 342 projecting from an upper surface 315 of the stem 314, as shown in Figure 8. Alternatively, the attachment portion 340 may take the form of a threaded bore for engagement with a complementary threaded attachment member 342. It will also be understood that other attachment means may be used to attach the insert 325 and stem 314, such as a bayonet type fitting, a press or push fit connector or other suitable attachment means. Alternatively, the stem may be bonded to the insert, or attached with an adhesive or moulded thereto.

It will also be understood that the device shown in Figure 3 may also be a two-part device with the stem 114 being attached directly to the femoral head 112. In this alternative, instead of the stem 114 attaching into an insert, as described in Figure 8, the stem 114 would attach directly into an attachment bore provided within the head portion 112. It will, of course, be understood that any of the methods of attachment discussed with regard to Figure 8 are also suitable if a separate stem were to be used in the embodiment of Figure 3.

In an alternative embodiment, the attachment portion 340 may take the form of a projection extending downwardly into the stem portion 314 from the top inner surface 316. The projection may be threaded or have a frustoconical shape or take other forms such as a bayonet fitting as described above. In this embodiment, the upper surface 315 of the stem, instead of merging with an upwardly extending attachment portion 342, would include a centrally positioned recess which is configured to engage with the projection depending from the top inner surface 316. The bore may be threaded or frustoconically shaped to engage with the projection provided from the top inner surface 316 of the insert 325 as appropriate.

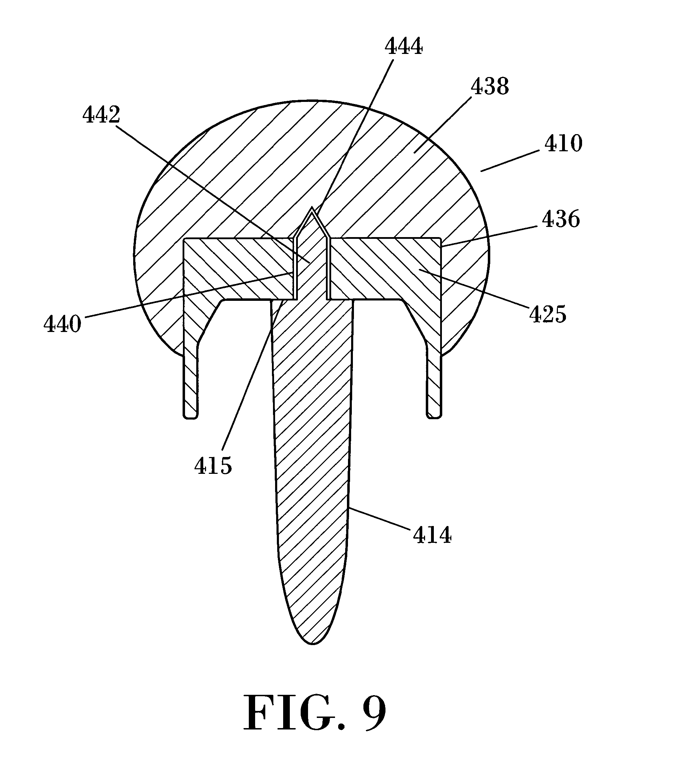

Figure 9 shows a yet further embodiment of the present invention which is a femoral head component 410 similar to that described with regard to Figure 8 and comprises an upper femoral head component 438, insert 425 and stem 414. The embodiment of Figure 9 differs to that of Figure 8 in that an attachment member 442 extends from an upper surface 415 of the stem 414 sufficiently for it to extend through a throughbore 440 provided in the insert 425 and into an attachment portion 444 provided in the upper femoral head component 438. The throughbore 440 may be frustoconically shaped to co-operate with a frustoconical attachment member 442 or threaded to correspond with a threaded attachment member 442. Other suitable attachment mechanisms, for example a bayonet fitting, may also be used. The femoral component 410 may be assembled by inserting the insert 425 into the insert recess 436, the attachment member 442 projecting from the stem 414 is then attached through the throughbore 440 into the attachment portion 442 in the upper femoral head component 438, thereby locking the separate parts of the femoral component 410 together. It will also be understood that an attachment projection may alternatively extend downwardly from the upper femoral component 438, through the throughbore 440 in the insert 425 and into a recess depending from the upper surface 415 of the stem 414.

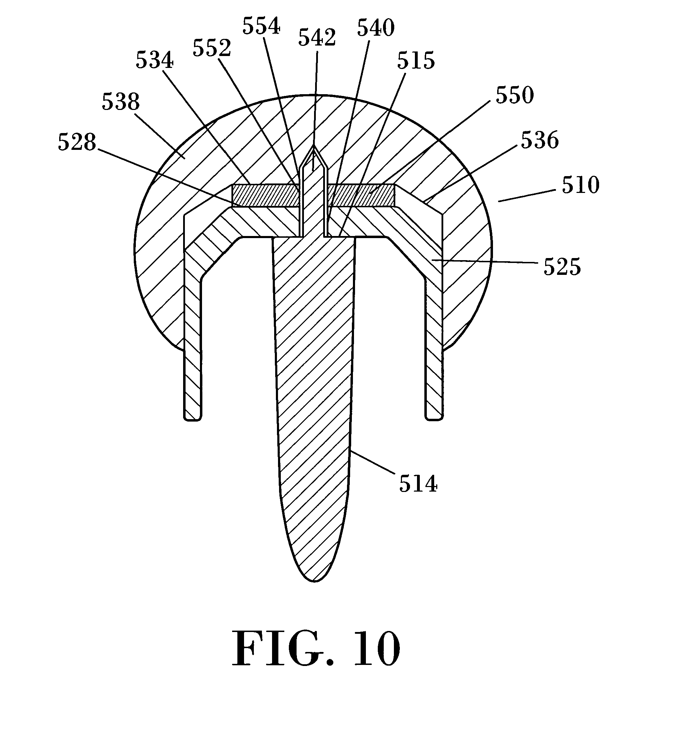

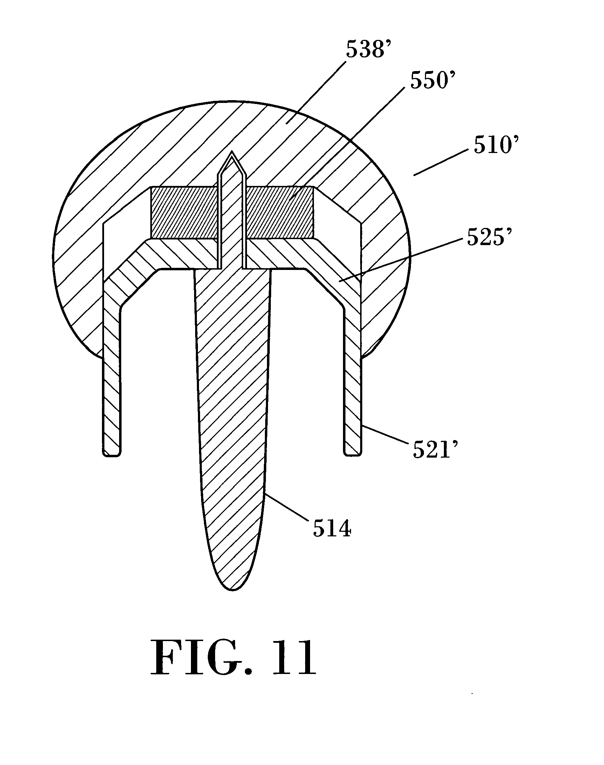

Figures 10 and 11 show a yet further embodiment of the present invention which is shown as being a four-part device. This embodiment is for a femoral head component 510 which has an insert receiving recess 536 as discussed previously in respect of Figures 7 to 9. An insert 525 similar to that previously discussed is insertable into the insert receiving recess 536. However, in contrast to the embodiment of Figure 9, a spacer 550 is placed between a top insert surface 528 and a top surface 534 of the insert receiving recess 536. Similarly to Figure 9, a stem 514 is provided with an attachment member 542 extending from an upper surface 515 of the stem 514. The insert 525 is provided with a throughbore 540, which may be of a complementary form to the attachment member 542. The attachment member 542 and throughbore 540 may take a number of complementary forms, for example complementary threads, being sized for press fitting, frustoconical shapes for taper attachment or a bayonet-type fixation. The spacer 550 is also provided with a throughbore 552, which may have the same complimentary form to that provided in the throughbore 540 of the insert 525. Finally a head portion bore 554 is provided into the upper femoral head component 538 itself. The femoral head component 510 is assembled, for example, by inserting the spacer 550 into the insert receiving recess 536 as appropriate, the insert 525 is then inserted into the insert receiving recess 536 and the attachment member 542 of the stem 514 is then inserted through the corresponding bores 540, 552 and into the bore 554 to lock the parts of the femoral head component 510 together. As can be seen in Figure 11, the use of a larger spacer 550' results in a longer skirt 521', thereby offsetting the centre of the prosthetic femoral head further from the femoral neck. It will, of course, be understood that the assembly of the femoral head component 510, 510' may differ from that described above. For example, the insert 550 may be firstly attached to the attachment member 542 before the insert 525 is entered into the recess 536. Furthermore, it will also be understood that the insert 525 and stem 514 may be provided as a single piece with a single attachment member 542 extending from the top insert surface 528. Furthermore, a spacer may be used with the embodiments of Figures 7 to 9, with the spacer being positioned between the insert and the upper head portion, and the insert then being attached to the upper femoral head component as previously discussed. The spacer may also take varying forms and may contact either a portion or the whole of the upper surfaces of the insert and the insert recess. Furthermore the insert may be attached to one or other of the insert or upper head portion, the insert then being attached to the upper head portion as described with regard to Figure 7. The various attachment mechanisms discussed previously may be utilized, for example a threaded attachment, a taper attachment or bayonet fitting. Bonding, adhesive and moulding of the various components is also envisaged.

Although the device in Figure 3 is suitable for the purposes of the invention it will be understood that the increasing modularity of the device as shown in Figures 7 to 11 and as described above allows for more interoperative choice for the surgeon. In particular looking at Figures 10 and 11 it can be seen that simply by the use of different spacers, different post-operative leg lengths can easily be achieved. Indeed, since a single head portion can be used with a variety of inserts and spacers there is a reduced inventory required for the very highly polished head portion.

It will also be appreciated that when the femoral head component is made more modular this means that different materials may be used. This may allow for significant cost savings and also improved workability when implanted. For example, a softer material may be used for the insert and skirt portion. Such a material may thereby cause less damage than the material needed for the bearing surface should there be any impingement between the skirt of the femoral component and the rim of the acetabulum.

Materials which are suitable for the various components of the femoral head prosthesis described above include stainless steel, cobalt chrome, titanium alloys, ceramic or other biocompatible materials. The insert and spacer, where applicable, alternatively may be wholly or partially made of a resorbable material such as polyglycolic acid, polylactic resorbable polymers, co-polymers of these or other known bio resorbable materials including metals such as magnezium alloys if desired. In any of the described embodiments all or part of the surface which attaches to a prepared femur may be provided with an osteo-conductive coating, such as hydroxyapetite, Titanium Plasma coating or metallic beaded type surfaces for use in a cementless implant procedure. Furthermore, any non-resorbable part of the stem, which in use will be located in the femur, may also be provided with an osteo-conductive coating to encourage fixation. Alternatively, it is understood that the implant may be fixed using a suitable bone cement.

The various modular implants discussed above may be implanted by firstly inserting the stem portion into the prepared femur and then attaching the insert and head portion as appropriate. Alternatively, the prosthesis may be assembled pre-operatively and then attached to the prepared femur by any suitable method.

In one example of the present invention, the diameter of the main head portion, comprising the bearing surface, is the same as the diameter of the outer surface of the skirt portion. An exemplary arrangement is the use of a 52mm diameter head with a 52mm diameter skirt. However, in order to provide a greater range of motion of the hip when using a device according to the present invention, the skirt may have a smaller diameter than that of the bearing surface, for example a 56mm diameter head with a 52mm diameter skirt.

Studies show that femoral components according to the present invention provide satisfactory range of motion of the hip joint in internal-external rotation, flexion-extension and abduction-adduction.

## Claims

1. A hip resurfacing femoral component for use with a prepared femur comprising:
a head portion having a substantially spherical outwardly facing bearing surface;
an attachment surface opposing said bearing surface of the head portion; and
a skirt portion having an outer skirt surface depending from said substantially spherical bearing surface and an inner skirt surface, the inner skirt surface and the attachment surface being configured to fit over the prepared femur.

2. A hip resurfacing femoral component for use with a prepared femur and having a distally extending longitudinal axis, said femoral component comprising,
a head portion having a substantially spherical distally facing bearing surface, having a centre and a distal-most point of the bearing surface; and
a proximally facing attachment surface opposing said bearing surface of the head portion and having a distal most point of the attachment surface;
an offset distance being defined as the distance parallel to the axis from said distal most point of the attachment surface to said distal most point of said bearing surface;
said femoral head being designed such that a ratio of the offset distance to the diameter of the substantially spherical bearing surface is greater than 0.25.

3. The hip resurfacing femoral component of claim 2 further comprising a skirt portion having an outer skirt surface depending from said substantially spherical bearing surface and an inner skirt surface, the inner skirt surface and the attachment surface being configured to fit over the prepared femur.

4. The hip resurfacing femoral component of any preceding claim further comprising a stem portion depending from said attachment surface for insertion into the prepared femur.

5. The resurfacing femoral component of any preceding claim comprising an insert portion, said insert portion providing said attachment surface and a head portion engagement surface, the head portion comprising an insert engagement surface opposing said bearing surface and for engagement with the head portion engagement surface of the insert portion.

6. The resurfacing femoral component of claim 5 wherein the head portion comprises an insert receiving recess bounded by the insert engagement surface and the skirt portion is provided as a separate portion, the skirt portion being held in position within and extending from said recess by the insert.

7. The resurfacing femoral component of claim 5 wherein said insert portion further provides the skirt portion with the inner skirt surface and the outer skirt surface.

8. The resurfacing femoral component of any of claims 5 to 7, wherein said head portion engagement surface and said insert portion engagement surface are attached by bonding, with adhesive or are moulded/fused together.

9. The resurfacing femoral component of any of claims 5 to 7, wherein said head portion engagement and insert portion engagement surfaces form a complementary frustoconical projection and recess combination to provide a taper fit between the head portion and the insert portion.

10. The resurfacing femoral component of any of claims 5 to 7, wherein said head portion engagement and insert portion engagement surfaces have complementary threads provided thereon to allow threaded engagement between the head portion and the insert portion.

11. The resurfacing femoral component of any of claims 5 to 7, wherein said head portion engagement and insert portion engagement surfaces are configured to provide a push or press fit engagement between the head portion and the insert portion.

12. The resurfacing femoral component of any of claims 5 to 11, further comprising a spacer to be positioned between the insert portion engagement surface and the head portion engagement surface.

13. The resurfacing femoral component of claim 12, wherein at least one of said insert portion engagement and head portion engagement surfaces is provided with engagement means for engagement with complementary engagement means provided on the spacer.

14. The resurfacing femoral component of any of claims 5 to 13, wherein the insert portion and any spacer have respective throughbores to accommodate a fixing member connecting the head portion to the stem portion.

15. The resurfacing femoral component of any preceding claim, wherein any of the head portion, any insert portion, any spacer or the stem portion are made of metal or ceramic.

16. The resurfacing femoral component of any preceding claim, wherein the attachment surface is coated with an osteo-conductive coating.

17. A method of implanting a resurfacing femoral component of any of claims 1 to 16, the method comprising:
providing the femoral component and assembling as necessary;
preparing the femur to receive a resurfacing femoral component;
placing the attachment surface of the resurfacing femoral component into engagement with the prepared femur.

18. A resurfacing femoral component substantially as herein described with reference to Figures 3 to 11.
